(19) Europäisches Patentamt
European Patent Office
Office européen des brevets

(11) Veröffentlichungsnummer: **0 040 357**
**B1**

(12) # EUROPÄISCHE PATENTSCHRIFT

(45) Veröffentlichungstag der Patentschrift: **19.09.84**

(51) Int. Cl.³: **C 07 D 249/04**

(21) Anmeldenummer: **81103434.7**

(22) Anmeldetag: **06.05.81**

(54) **Verfahren zur Herstellung von v-Triazolyl-(2)-phenolen.**

(30) Priorität: **17.05.80 DE 3018963**

(43) Veröffentlichungstag der Anmeldung:
**25.11.81 Patentblatt 81/47**

(45) Bekanntmachung des Hinweises auf die Patenterteilung:
**19.09.84 Patentblatt 84/38**

(84) Benannte Vertragsstaaten:
**CH DE FR GB IT LI**

(56) Entgegenhaltungen:
**DE - A - 2 210 261**
**DE - A - 2 242 784**
**DE - A - 2 848 670**
**FR - A - 1 559 131**

**Austr. J. Chem. 22 (1969), 1915**
**Neuere Methoden der präparativen Chemie Band V S. 54**
**Chemistry of Carbon Compounds (1957) Vol IV, Part A S.439-49**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Dorlars, Alfons, Dr., Mozartstrasse 32, D-5090 Leverkusen (DE)**
Erfinder: **Schroeder, Josef, Dr., Paul-Klee-Strasse 64, D-5090 Leverkusen (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung ist ein verbessertes Verfahren zur Herstellung von 3-(v-Triazolyl-2')-phenolen, welche wichtige Zwischenprodukte zur Herstellung von wertvollen optischen Aufhellern der Cumarinreihe darstellen.

Es ist bereits bekannt, die Titelverbindungen durch Diazotieren entsprechender Triazolylaniline und anschließendes Verkochen der Diazoniumgruppe herzustellen (vgl. DE-A-2 848 670).

Diese Methode weist jedoch eine Reihe von Nachteilen auf, wie geringe Ausbeuten, aufwendige Reinigungsoperationen und Schwerzugänglichkeit der Ausgangsmaterialien.

Es wurde gefunden, daß man die genannten Triazolylphenole der Formel I

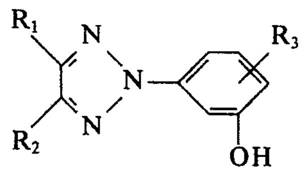

(I)

im technischen Maßstab in einfacher Weise und hohen Ausbeuten herstellen kann, wenn man entweder 3-(v-Triazolyl-2')-benzolsulfonsäuren der Formel II

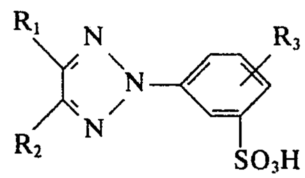

(II)

bzw. deren Alkalisalze, worin

$R_1$ und $R_2$ unabhängig voneinander Alkyl, Aralkyl oder gegebenenfalls durch $C_1-C_4$-Alkyl, $C_1-C_4$-Alkoxy oder Phenyl substitiertes Phenyl,
$R_3$ Wasserstoff oder Alkyl bedeuten,

oder Oximinohydrazone der Formel III

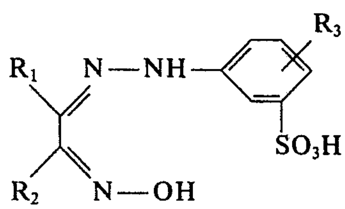

(III)

bzw. deren Alkalisalze — gegebenenfalls in Gegenwart von katalytischen Mengen an Kupfer oder Kupferverbindungen sowie gegebenenfalls in Anwesenheit von Natrium- oder Kaliumacetat, Erdalkalimetallhydroxiden oder Natriumaluminat — bei 200—300° C einer Alkalischmelze unterwirft.

Die Herstellung von Phenolen durch »Alkalischmelzen« ist an sich nichts Neues, dennoch muß es als ausgesprochen überraschend angesehen werden, daß diese im Grunde sehr drastische Methode hier so glatt verläuft und vorzügliche Ausbeuten liefert, da aus »Neuere Methoden der präparativen organischen Chemie V, S. 54« bekannt war, daß Azolreste — ähnlich wie die Halogenatome in Säurehalogeniden — abspaltbare Gruppen sind und daher erwartet werden konnte, daß unter den vorstehend genannten Reaktionsbedingungen unerwünschte Nebenreaktionen (z. B. Ringabspaltung) eintreten würde.

In den obengenannten Formeln sind geeignete Alkylreste solche mit 1—4 C-Atomen.

Geeignete Aralkylreste sind Phenyl-$C_1-C_3$-alkylreste.

Die Verwendung der Sulfonsäuren der Formel II erweist sich naturgemäß im allgemeinen als günstiger hinsichtlich der stöchiometrischen Ausbeuten; jedoch kann bei bestimmter Substitution (z. B. $R_1$ und $R_2$ = Niedrigalkyl) und im Hinblick auf die Wirtschaftlichkeit des gesamten Syntheseweges der Einsatz der α-Oximino-phenylhydrazonsulfonsäuren III vorzuziehen sein, wobei häufig der Zusatz katalytischer Mengen an Kupfer, bzw. Kupferverbindungen vorteilhaft ist.

Die v-Triazolyl-benzolsulfonsäuren II können nach verschiedenen bekannten Verfahren durch Cyclodehydratisierung der entsprechenden α-Oximino-phenylhydrazonsulfonsäuren III hergestellt werden, z. B. durch Einwirkung von Acylierungsmitteln, wie z. B. Acetanhydrid, in Gegenwart von Basen und katalytischen Mengen Kupfer oder Kupferverbindungen oder von Harnstoff in Wasser oder Lösungsmitteln (vgl. die oben zitierte Patentliteratur).

Die α-Oximino-phenylhydrazonsulfonsäuren III ihrerseits sind in technisch vorteilhafter Weise

durch Kondensation von $\alpha$-Oximinoketonen IV mit Phenylhydrazinsulfonsäuren V erhältlich:

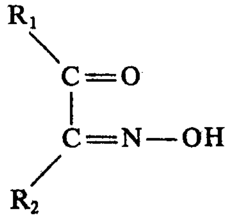

+

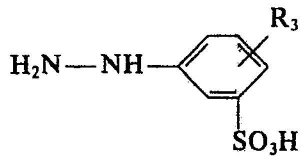

(IV) (V)

$R_1$—$R_3$ haben die obengenannte Bedeutung.

Als Alkalihydroxide kommen besonders Natrium- und Kaliumhydroxid in Betracht, sowie Mischungen beider Hydroxide. Da man eine möglichst niedrige Reaktionstemperatur anstreben wird, sind in der Nähe des Eutektikums liegende Mischungsverhältnisse günstig, die eine gute Rührbarkeit der Reaktionsmischung gewährleisten. Auch Zusatzstoffe, wie Natrium- oder Kaliumacetat, Erdalkalimetallhydroxide, Natriumaluminat, sowie geringe Mengen an Wasser können anwesend sein, um die Eigenschaften der Schmelze günstig zu beeinflussen. Man kann die Alkalihydroxide auch als wäßrige Lösungen einsetzen und das Wasser vor oder nach Eintragen der Sulfonsäure II, bzw. III in dem gewünschten Maße herausdestillieren, bevor die Reaktionstemperatur erreicht wird.

Die Umsetzung ist durchweg in weniger als 1 Stunde beendet. Geringe Mengen an abgespaltenem v-Triazol können entweder in einem Arbeitsgang oder in einer folgenden Extraktion durch eine Säurebehandlung (z. B. mit Salzsäure) entfernt werden.

Das Verfahren sei anhand der folgenden Beispiele näher erläutert.

## Beispiel 1

### 3-[4-Phenyl-5-methyl-v-triazolyl-(2)]-phenol

In einem 5 l-Niederdruck-Rührkessel werden bei 250° C unter Normaldruck 1,13 kg Natriumhydroxid und 200 g Kaliumhydroxid (techn.) unter Rühren verschmolzen. Bei dieser Temperatur werden 2,03 kg (6 Mol) trockenes Natriumsalz der 3-[4-Phenyl-5-methyl-v-triazolyl-(2)]-benzolsulfonsäure innerhalb von 30 Minuten unter Rühren eingetragen. Man rührt 10 Minuten bei 250° und 30 Minuten bei 275—280°, läßt dann auf 265° abkühlen und pumpt dann nach Schließen der Ventile 2,6 l Wasser in der Weise in die Schmelze, daß ein Druck von 5 bar nicht überschritten wird. Man läßt unter weiterem Rühren auf 100° abkühlen, drückt die Lösung in einen 15 l-Kessel und läßt dann bei etwa 70—80° 3,5 l Salzsäure d = 1,18 unter die Oberfläche zutropfen. Man rührt noch $^1/_2$ Stunde bei 90—95° bei stark kongosaurer Reaktion nach, wodurch geringe Mengen an 4-Phenyl-5-methyl-v-triazol in Lösung gehen. Schließlich saugt man das ausgefallene helle Triazolylphenyl ab, wäscht es auf der Nutsche mit wenig 5%iger Salzsäure, dann mit 10 l Wasser aus und trocknet es. Man erhält 1,21 kg (80,4% der Theorie) an hellem kristallinem 3-[4-Phenyl-5-methyl-v-triazolyl-(2)]-phenol vom Schmelzpunkt 152—153°.

Durch Neutralisieren der sauren Filtrate erhält man 87 g (ca. 9% der Theorie) 4-Phenyl-5-methyl-v-triazol vom F. 173°.

Verwendet man anstelle der 3-[4-Phenyl-5-methyl-v-triazolyl-(2)]-benzolsulfonsäure gleichmolare Mengen an 3-[4-p-Biphenylyl-5-methyl-v-triazolyl-(2)]-benzolsulfonsäure und verfährt im übrigen in analoger Weise, so erhält man in 76%iger Ausbeute das 3-[4-p-Biphenylyl-5-methyl-v-triazolyl-(2)]-phenol in Form heller Kristalle vom Schmelzpunkt 187—188°.

Setzt man in analoger Weise 3-[4,5-Diphenyl-v-triazolyl-(2)]-benzolsulfonsäure ein und verfährt wie oben angegeben, so erhält man das 3-[4,5-Diphenyl-v-triazolyl-(2)]-phenol, helle Kristalle vom Schmelzpunkt 128—130° (Ausbeute ca. 65%).

In entsprechender Weise läßt sich aus 5-[4-Phenyl-5-methyl-v-triazolyl-(2)]-methylbenzolsulfonsäure in 85%iger Ausbeute das 5-[4-Phenyl-5-methyl-v-triazolyl-(2)]-2-methylphenol als farblose Kristalle vom Schmelzpunkt 173—173,5° erhalten.

## Beispiel 2

### 3-[4-Ethyl-5-methyl-v-triazolyl-(2)]-phenol

565 g Phenylhydrazin-3-sulfonsäure werden in 1,5 l Wasser mit 346 g Oximinodiethylketon verrührt, wobei man durch Zugabe von Natronlauge einen pH-Wert von 5,0—5,5 dauernd einhält und die Temperatur auf 50—55° bringt. Nach etwa 2-stündigem Rühren ist die Phenylhydrazinsulfonsäure

verbraucht; man stellt dann mit Natronlauge auf pH 7. Darauf salzt man bei 10° mit 80 g Kochsalz aus, saugt das ausgeschiedene Natriumsalz der Oximino-diethylketon-phenylhydrazonsulfonsäure ab und trocknet es bis zur Gewichtskonstanz. Das so erhaltene Oximinohydrazonsulfonat trägt man allmählich in einen Niederdruck-Rührkessel ein, in dem 326 g technisches Natriumhydroxid und 370 g Kaliumhydroxid mit 125 ml Natronlauge 46° Bé bei 190° verschmolzen wurden. Gleichzeitig werden 6 g Kupferpulver zugegeben. Sobald alles eingetragen ist, wird der Kessel geschlossen und etwa eine Stunde auf 240° gehalten. Schließlich läßt man auf 210° abkühlen, pumpt 1 l Wasser in die Schmelze, läßt unter Rühren auf 95° abkühlen, gibt 15 g Adsorptionskohle hinzu und klärt die alkalische Lösung. In das Filtrat tropft man unter Kühlung 1,8 l Salzsäure d = 1,18; bei einer Endtemperatur von ca. 45—50° läßt man das abgeschiedene rohe, ölige Triazolylphenol absitzen, trennt es ab und unterwirft es nach Abdestillieren von etwas Wasser einer fraktionierenden Destillation. Bei einem Druck von 0,5 mbar gehen bei 146° 365 g (ca. 60% der Theorie, bezogen auf eingesetzte Phenylhydrazinsulfonsäure) 3-[4-Ethyl-5-methyl-v-triazolyl-(2)]-phenol als farbloses Öl über, das in der Vorlage allmählich kristallin erstarrt.

In analoger Weise und mit vergleichbaren Ausbeuten erhält man aus 3-Oximino-heptanon-(4), bzw. Diacetylmonoxim und Phenylhydrazin-3-sulfonsäure das 3-[4-n-Propyl-5-ethyl-v-triazolyl-(2)]-phenol, bzw. das 3-[4,5-Dimethyl-v-triazolyl-(2)]-phenol.

Nimmt man eine weitere Synthesestufe in Kauf und setzt das Oximinodiethylketon-phenylhydrazonsulfonat zunächst in bekannter Weise zur 3-[4-Ethyl-5-methyl-v-triazolyl-(2)]-benzolsulfonsäure (Natriumsalz) um und unterwirft dieses einer einstündigen Schmelze mit 440 g Natriumhydroxid und 330 g Kaliumhydroxid bei 240°, so erhält man das 3-[4-Ethyl-5-methyl-v-triazolyl-(2)]-phenol in Ausbeuten von ca. 75%, bezogen auf die eingesetzte v-Triazolylbenzolsulfonsäure.

## Patentanspruch

Verfahren zur Herstellung von 3-(v-Triazolyl-2')-phenolen der Formel I

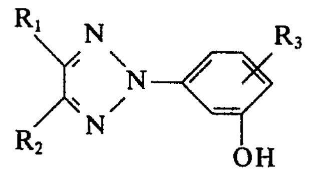

(I)

dadurch gekennzeichnet, daß man

a) 3-(v-Triazolyl-2')-benzolsulfonsäuren der Formel II

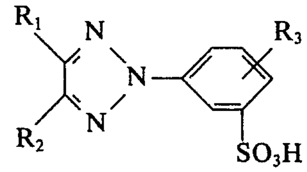

(II)

bzw. deren Alkalisalze, worin

$R_1$ und $R_2$ unabhängig voneinander Alkyl, Aralkyl oder gegebenenfalls durch $C_1$—$C_4$-Alkyl, $C_1$—$C_4$-Alkoxy oder Phenyl substituiertes Phenyl und
$R_3$ Wasserstoff oder Alkyl bedeuten, oder

b) Oximinohydrazone der Formel III

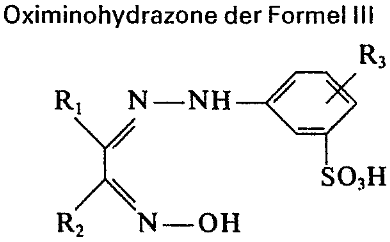

(III)

bzw. deren Alkalisalze — gegebenenfalls in Gegenwart von katalytischen Mengen an Kupfer oder Kupferverbindungen sowie gegebenenfalls in Anwesenheit von Natrium- oder Kaliumacetat, Erdalkalimetallhydroxiden oder Natriumaluminat — bei 200—300° C einer Alkalihydroxidschmelze unterwirft.

**Claim**

Process for the preparation of 3-(v-triazol-2'-yl)-phenols of the formula I

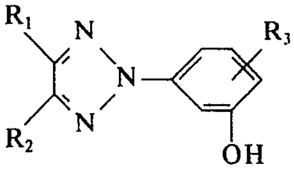

(I)

characterised in that

a) 3-(v-triazol-2'-yl)-benzenesulphonic acids of the formula II

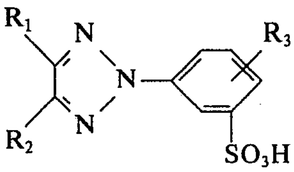

(II)

or alkali metal salts thereof wherein

$R_1$ and $R_2$ independently of one another denote alkyl, aralkyl or phenyl which is optionally substituted by $C_1-C_4$-alkyl, $C_1-C_4$-alkoxy or phenyl and
$R_3$ denotes hydrogen or alkyl, or

b) oximinohydrazones of the formula III

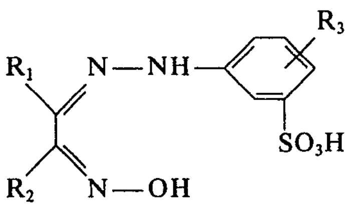

(III)

or alkali metal salts thereof are subjected to fusion with an alkali metal hydroxide at 200—300° C — optionally in the presence of catalytic amounts of copper or copper compounds and optionally in the presence of sodium acetate or potassium acetate, alkaline earth metal hydroxides or sodium aluminate.

**Revendication**

Procédé pour la fabrication de 3-(v-triazolyl-2')-phénols de formule I

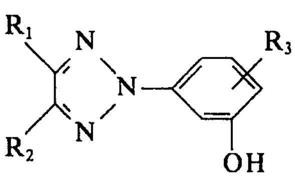

(I)

caractérisé en ce que l'on soumet

(a) des acides 3-(v-triazolyl-2')-benzènesulfonique de formule II

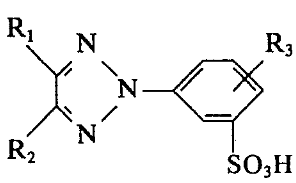

(II)

dans laquelle

$R_1$ et $R_2$ représentent indépendamment l'un de l'autre un groupe alkyle ou arylalkyle ou un groupe phényle éventuellement substitué par alkyle en $C_1-C_4$, alcoxy en $C_1-C_4$ ou phényle et

$R_3$ représente l'hydrogène ou un alkyle,

ou leurs sels alcalins, ou

(b) des oximinohydrazones de formule III

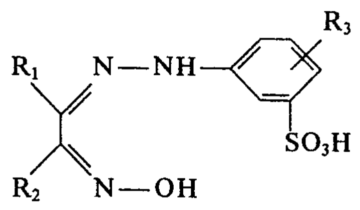

(III)

on leurs sels alcalins à une fusion à 200—300°C avec un hydroxyde alcalin — éventuellement en présence de quantités catalytiques de cuivre ou de composés de cuivre, ainsi éventuellement qu'en présence d'acétate de sodium ou de potassium, d'hydroxydes de métaux alcalinoterreux ou d'aluminate de sodium.